(19) Europäisches Patentamt
European Patent Office
Office européen des brevets

(11) EP 4 756 046 A1

(12) EUROPEAN PATENT APPLICATION
published in accordance with Art. 153(4) EPC

(43) Date of publication:
10.06.2026 Bulletin 2026/24

(51) International Patent Classification (IPC):
C22B 23/00 (2006.01)    G16C 60/00 (2019.01)

(21) Application number: 24939062.6

(22) Date of filing: 30.09.2024

(86) International application number:
PCT/CN2024/122776

(87) International publication number:
WO 2026/065407 (02.04.2026 Gazette 2026/14)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(71) Applicants:
• GEM Co., Ltd.
Shenzhen, Guangdong 518100 (CN)
• PT ESG New Energy Material
Jakarta Selatan, Provinsi DKI Jakarta 12950 (ID)
• PT Green Eco Nickel
Jakarta Selatan, Provinsi DKI Jakarta, 12950 (ID)
• PT QMB New Energy Materials
Jakarta Selatan, Provinsi DKI Jakarta, 12950 (ID)

(72) Inventors:
• Xu, Kaihua
Shenzhen, Guangdong 518100 (CN)
• Hasibuan, Andi Syaputra
Jakarta Selatan,
Provinsi DKI Jakarta, 12950 (ID)
• Aji, Tegar Mukti
Jakarta Selatan,
Provinsi DKI Jakarta, 12950 (ID)
• Wanaldi, Rizky
Jakarta Selatan,
Provinsi DKI Jakarta, 12950 (ID)

(74) Representative: Tiburzi, Andrea et al
Barzanò & Zanardo Roma S.p.A.
Via Piemonte 26
00187 Roma (IT)

(54) METHOD AND PROCESS FOR PREPARING MHP BY CONTROLLING CRYSTALLIZATION IN HYDROMETALLURGY OF LATERITE NICKEL ORE

(57) Provided are a method and a process for preparing MHP via controlled crystallization in hydrometallurgical processing of laterite nickel ore, belonging to the technical field of laterite nickel ore hydrometallurgy. The method comprises: determining a nuclei count of multiple crystal nuclei after nucleation reaction of a solution after iron and aluminum removal, based on nucleation reaction conditions; determining an average particle size of multiple crystal grains after primary growth of the multiple crystal nuclei, based on primary growth reaction conditions; and determining a predicted particle size distribution of agglomerated crystal grains after secondary agglomeration and growth of the multiple crystal grains, based on the Metropolis algorithm, the nuclei count, and the average particle size. According to the predicted particle size distribution results, the present application adjusts reaction conditions for the nucleation reaction and primary growth reaction to control the nuclei count and average particle size, thereby obtaining high-performance MHP products with optimized particle size distribution, providing guiding significance for MHP crystallization processes.

FIG. 1

**Description**

TECHNICAL FIELD

The present application relates to the technical field of laterite nickel ore hydrometallurgy, and specifically relates to a method and a process for preparing MHP via controlled crystallization in hydrometallurgical processing of laterite nickel ore.

BACKGROUND

Nickel-cobalt-manganese hydroxide (MHP) is an intermediate product of nickel prepared by high-pressure acid leaching technology with laterite nickel ore as the raw material. Nickel-cobalt-manganese hydroxide can be used in the production of nickel sulfate, refined nickel-cobalt-manganese hydroxide, nickel plate and other products, especially for the production of nickel sulfate, which is one of the main materials of cathode materials for ternary batteries. In recent years, with the continuous development of electric vehicles and other industries, the market capacity of ternary batteries has continued to expand, which promotes the market demand for nickel-cobalt-manganese hydroxide.
The preparation process of the nickel-cobalt-manganese hydroxide is as follows: adding a precipitating agent to a solution after iron and aluminum removal, followed by nucleation, primary crystal growth, and secondary agglomeration/-growth of nickel-cobalt-manganese hydroxide from the solution, ultimately yielding MHP. The particle size distribution of agglomerated crystal grains obtained after secondary agglomeration/growth significantly impacts MHP performance. Conventional techniques evaluate the particle size distribution of experimentally obtained agglomerates to determine their properties but cannot predict the particle size distribution of agglomerated crystals. Consequently, reaction conditions cannot be optimized before production to achieve the required particle size distribution, leading to uncontrollable MHP performance. In practical production, ideal MHP products should exhibit a narrow particle size distribution range and uniform morphology. Such precipitate characteristics enable high settling rates, fast filtration speeds, and low filter cake moisture content.
Therefore, there is an urgent need to provide a method and process for preparing MHP via controlled crystallization in hydrometallurgical processing of laterite nickel ore to accurately predict the PSD of agglomerated crystals. This will ensure MHP products possess a narrow particle size distribution, thereby enhancing the performance of the prepared nickel-cobalt-manganese hydroxide.

SUMMARY

In view of the above, it is necessary to provide a method and a process for preparing MHP via controlled crystallization in hydrometallurgical processing of laterite nickel ore, to solve the technical problems in the existing art that the particle size distribution of the agglomerated grains cannot be predicted before preparation, thus reaction conditions cannot be optimized, resulting in the uncontrollable properties of the prepared nickel-cobalt-manganese hydroxide.
In one aspect, in order to solve the above technical problems, the present application provides a method for preparing MHP via controlled crystallization in hydrometallurgical processing of laterite nickel ore, including:

determining a nuclei count of multiple crystal nuclei after nucleation reaction of a solution after iron and aluminum removal, based on nucleation reaction conditions;

determining an average particle size of multiple crystal grains after primary growth of the multiple crystal nuclei, based on primary growth reaction conditions;

determining a predicted particle size distribution of agglomerated crystal grains after secondary agglomeration and growth of the multiple crystal grains, based on the Metropolis algorithm, the nuclei count, and the average particle size.

In one possible embodiment, the nucleation reaction condition includes pH and mass of a precipitating agent; and the determining the nuclei count of multiple crystal nuclei after nucleation reaction of a solution after iron and aluminum removal based on nucleation reaction conditions comprises:

determining a supersaturation degree of a primary nickel-cobalt-manganese hydroxide precipitation solution based on the pH and mass of the precipitating agent;

determining a crystallization rate of the primary nickel-cobalt-manganese hydroxide precipitation solution based on the supersaturation degree and a crystallization rate model;

determining the nuclei count of the multiple crystal nuclei based on the crystallization rate.

In one possible embodiment, the crystallization rate model is as follows:

$$J = J_0 \exp\left(\frac{-16\pi\gamma^3 \overline{V}^2}{3k_B R_g^2 T^3 (\ln S)^2}\right)$$

$$J_0 = A \cdot \ln S$$

$$S = c_{M^{2+}} * c_{OH}^2 / Ksp_{M(OH)_2}$$

$$A = \frac{1}{V} \cdot \left(\frac{N_A R_g T}{\gamma}\right) 0.5 D_{M^{2+}} (Ksp_{M(OH)_2})^{1/3}$$ ;

wherein $J$ refers to crystallization rate, $1/(m^3 \cdot s)$; $J_0$ refers to maximum crystallization rate, $1/(m^3 \cdot s)$; $\gamma$ refers to surface energy of hydroxide precipitate, $J/m^2$; $\overline{V}$ refers to partial molar volume of hydroxide, $mol/m^3$; $k_B$ refers to Boltzmann's constant; $R_g$ refers to ideal gas constant, $J/(mol \cdot K)$; $T$ refers to temperature, K; $S$ refers to supersaturation degree; $A$ refers to proportional constant; $c_{M^{2+}}$ refers to a concentration of divalent ions in a solution after iron and aluminum removal; $c_{OH}$ refers to the concentration of hydroxide ions; $Ksp_{M(OH)_2}$ refers to solubility product of hydroxide precipitate; $N_A$ refers to Avogadro's constant; and $D_{M^{2+}}$ refers to diffusion rate constant of $M^{2+}$ metal ions in a solution.

In one possible embodiment, the determining the average particle size of multiple crystal grains after primary growth of the multiple crystal nuclei based on primary growth reaction conditions comprises:

determining a hydroxide precipitation concentration during primary growth of the crystal nuclei based on the primary growth reaction conditions;

determining the average particle size of the multiple crystal grains based on the hydroxide precipitation concentration, a partial differential equation for crystal grain growth, and a boundary condition.

In one possible embodiment, the partial differential equation for crystal grain growth is as follows:

$$\frac{\partial C_{M(OH)_2}}{\partial t} = D \frac{\partial^2 C_{M(OH)_2}}{\partial r^2} + \frac{2D}{r} \frac{\partial C_{M(OH)_2}}{\partial r}$$ ;

and the boundary condition is as follows:

$$C_{M(OH)_2} \frac{dR}{dt} = -D \frac{\partial C_{M(OH)_2}}{\partial r}\bigg|_{r=R}$$

$$C_{M(OH)_2}(r=R) = \breve{C}_{M(OH)_2}$$ ;

wherein $C_{M(OH)_2}$ refers to a concentration of hydroxide precipitate after the primary growth reaction condition; D refers to diffusion rate constant of hydroxide precipitate in a solution; r refers to vector position, t refers to time; c(r,t) refers to component field variable; R refers to maximum position of vector field; $\breve{C}_{M(OH)_2}$ refers to a concentration of hydroxide precipitate in the solution phase.

In one possible embodiment, the determining a predicted particle size distribution of agglomerated crystal grains after secondary agglomeration and growth of the multiple crystal grains based on the Metropolis algorithm, the nuclei count and the average particle size comprises:

step 1: setting the nuclei count and the average particle size as an initial state;

step 2: exchanging positions of the liquid and crystal grains in the nickel-cobalt-manganese hydroxide precipitation solution during the secondary agglomeration and growth;

step 3: determining total energy of the nickel-cobalt-manganese hydroxide precipitation solution after exchanging the positions of liquid and crystal grains;

step 4: determining a variation trend of the total energy: accepting position changes when the trend indicates decreasing energy; accepting position changes with a predetermined probability when the trend indicates increasing energy;

step 5: returning to Step 2 and repeating Steps 2 to 4 until a count of position changes reaches a predetermined iteration count, then using final positions of liquid and crystal grains as target positions; and

step 6: determining the predicted particle size distribution of the agglomerated crystal grains based on the target positions.

In one possible embodiment, the total energy includes agglomeration energy and stirring energy.

In one possible embodiment, the maximum distance between the exchanged-position liquid and crystal grains satisfies:

$$\Delta x_{max} = \sqrt{D_p \times \Delta t},$$

wherein $\Delta x_{max}$ refers to the maximum distance; $D_p$ refers to the diffusion rate of crystal grains, $m^2/s$; $\Delta t$ refers to the time interval between two consecutive iterative calculations.

In one possible embodiment, the method further includes:

when the predicted particle size distribution fails to satisfy a desired particle size distribution, optimizing the nucleation reaction conditions and/or the primary growth reaction conditions until the predicted particle size distribution satisfies the desired particle size distribution.

In another aspect, the present application provides a process for preparing MHP via controlled crystallization in hydrometallurgical processing of laterite nickel ore, which is achieved based on the method for preparing MHP via controlled crystallization in hydrometallurgical processing of laterite nickel ore;

wherein the method for preparing MHP via controlled crystallization in hydrometallurgical processing of laterite nickel ore is based on the method for preparing MHP via controlled crystallization in hydrometallurgical processing of laterite nickel ore as described in any one of the above possible embodiments.

The beneficial effects of the present application are as follows. The method for preparing MHP via controlled crystallization in hydrometallurgical processing of laterite nickel ore disclosed in the present application first determines a nuclei count of multiple crystal nuclei after nucleation reaction of a solution after iron and aluminum removal based on nucleation reaction conditions, then determines an average particle size of multiple crystal grains after primary growth of the nuclei based on primary growth reaction conditions. By using the nuclei count and average particle size as prerequisite parameters for predicting particle size distribution, the accuracy of the determined predicted particle size distribution is enhanced.

Further, due to the randomness of secondary agglomeration and growth of grains, the predicted particle size distribution is determined based on the Metropolis algorithm in the present application; by utilizing characteristics of simulating random sampling distribution of the Metropolis algorithm, the purpose of accurately describing the processes of secondary agglomeration and growth of grains can be achieved, further improving the accuracy of the predicted grain size distribution.

In the present application, based on the results of the predicted particle size distribution, the reaction condition for nucleation reaction and primary growth reaction is adjusted to control the nuclei count and the average particle size of crystal nuclei, and high-performance MHP products with better particle size distribution can be obtained, which has a guiding role in the crystallization process of MHP.

BRIEF DESCRIPTION OF DRAWINGS

In order to explain the technical solution in the embodiments of the present application more clearly, the drawings needed in the description of the embodiments will be briefly introduced below. Obviously, the drawings described below are only some embodiments of the present application. For those skilled in the art, other drawings can be obtained according to these drawings without creative work.

FIG. 1 is a flow chart of an embodiment of the method for preparing MHP via controlled crystallization in hydrometallurgical processing of laterite nickel ore provided by the present application;

FIG. 2 is a flow chart of an embodiment of S 101 in FIG. 1 of the present application;

FIG. 3 is a flow chart of an embodiment of S 102 in FIG. 1 of the present application;

FIG. 4 is a flow chart of an embodiment of S103 in FIG. 1 of the present application;

FIG. 5 is a schematic diagram of the predicted particle size distribution corresponding to different pH of the precipitating agent provided by the present application; and

FIG. 6 is a comparison diagram of the particle size distribution before and after optimization provided by the present application.

DETAILED DESCRIPTION

The technical solution in the present application will be described clearly and completely in conjunction with attached figures in the specific embodiments of the present application. Apparently, the embodiments described are part, not all, of the embodiments of the present application. Based on the embodiments of the present application, all other embodiments obtained by those skilled in the art are within the protective scope of the present application on the premise that no creative work is done.

It should be understood that the illustrative figures are not drawn to scale. The flow chart used in the present application illustrates the operations carried out according to some embodiments of the present application. It should be understood that the operations in the flowchart can be performed out of order, and steps that do not have a logical contextual relationship can be reversed or executed simultaneously. Furthermore, those skilled in the art, guided by the content of the present application, can add one or more additional operations to the flowchart or remove one or more operations from the flowchart. Some of the block diagrams shown in the figures are functional entities and do not necessarily correspond to physically or logically independent entities. These functional entities can be implemented in software form, or in one or more hardware modules or integrated circuits, or in different networks and/or processor systems and/or microcontroller systems.

The term "embodiment" herein refers to specific features, structures, or characteristics described in conjunction with the embodiment may be included in at least one embodiment of the present application. The phrase appearing in various positions in the manual does not necessarily refer to the same embodiment, nor an independent or alternative embodiment that is mutually exclusive with other embodiments. Those skilled in the art should explicitly and implicitly understand that the embodiments described herein can be combined with other embodiments.

The present application provides a method and a process for preparing MHP via controlled crystallization in hydrometallurgical processing of laterite nickel ore, which will be illustrated separately below.

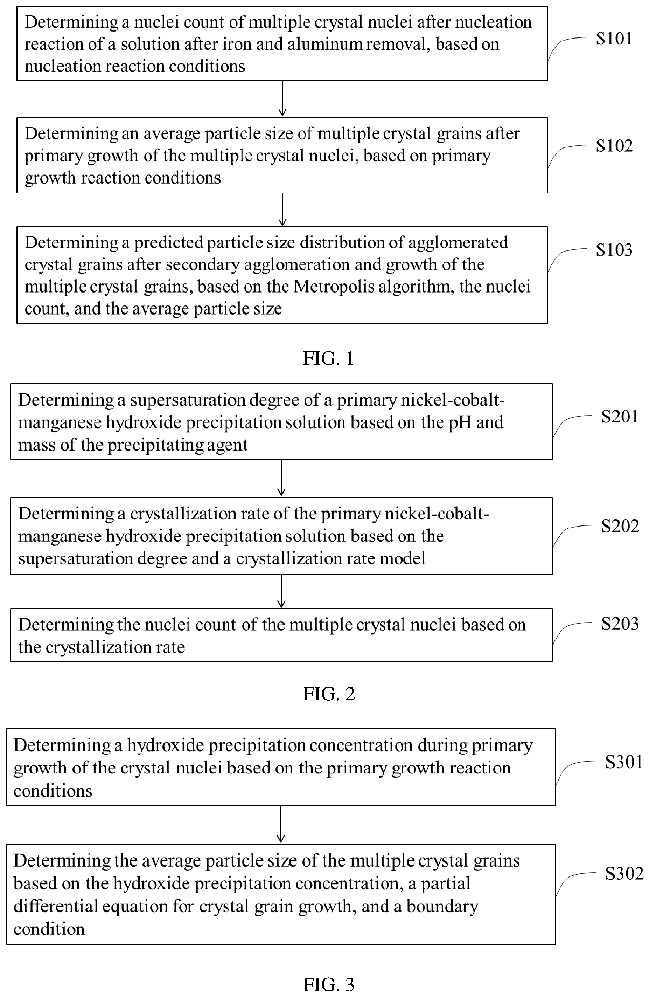

FIG. 1 is a flow chart of an embodiment of the method for preparing MHP via controlled crystallization in hydrometallurgical processing of laterite nickel ore provided by the present application. As shown in FIG. 1, the method for preparing MHP via controlled crystallization in hydrometallurgical processing of laterite nickel ore includes:

S101: determining a nuclei count of multiple crystal nuclei after nucleation reaction of a solution after iron and aluminum removal, based on nucleation reaction conditions;

S102: determining an average particle size of multiple crystal grains after primary growth of the multiple crystal nuclei, based on primary growth reaction conditions; and

S103: determining a predicted particle size distribution of agglomerated crystal grains after secondary agglomeration and growth of the multiple crystal grains, based on the Metropolis algorithm, the nuclei count, and the average particle size.

In step S101, the solution after iron and aluminum removal is the solution obtained from high-pressure acid leaching and iron-aluminum removal of laterite nickel ore.

It should be noted that both the nucleation reaction and the primary growth reaction involve adding a precipitating agent to the solution, and a precipitation reaction of the seed crystals is performed based on the addition of the precipitating agent, achieving crystal nucleus formation and grain growth.

It should be understood that the precipitating agent is at least one of sodium hydroxide, lime milk, potassium hydroxide, calcium oxide, magnesium oxide, and magnesium hydroxide.

Compared with the prior art, in the method for preparing MHP via controlled crystallization in hydrometallurgical processing of laterite nickel ore provided in the present application, the nuclei count of multiple crystal nuclei after the nucleation reaction of the solution after iron and aluminum removal is firstly determined based on the nucleation reaction condition; then, the average particle size of multiple grains after primary growth of the multiple crystal nuclei is determined based on the primary growth reaction condition; the nuclei count and the average particle size are used as prerequisite parameters for predicting the particle size distribution, which can improve the accuracy of the predicted particle size distribution.

Further, due to the randomness of secondary agglomeration and growth of grains, the predicted particle size distribution is determined based on the Metropolis algorithm in the present application; by utilizing characteristics of simulating random sampling distribution of the Metropolis algorithm, the purpose of accurately describing the processes of secondary agglomeration and growth of grains can be achieved, further improving the accuracy of the predicted grain size distribution.

In some embodiments of the present application, the condition for the nucleation reaction and primary growth reaction includes at least one parameter that affects the nucleation and the primary growth, for example, including a parameter of a precipitating agent, and/or a parameter of an solution after iron and aluminum removal; the parameter of the precipitating agent includes mass and pH of the precipitating agent, and the parameter of the solution after iron and aluminum removal includes mass and concentration of the solution after iron and aluminum removal.

In a specific embodiment of the present application, the condition for the nucleation reaction includes pH and mass of the precipitating agent; as shown in FIG. 2, the step S101 includes:

S201: determining a supersaturation degree of a primary nickel-cobalt-manganese hydroxide precipitation solution based on the pH and mass of the precipitating agent;

S202: determining a crystallization rate of the primary nickel-cobalt-manganese hydroxide precipitation solution based on the supersaturation degree and a crystallization rate model; and

S203: determining the nuclei count of the multiple crystal nuclei based on the crystallization rate.

The embodiment of the present application determines the crystallization rate through an effective and accurate crystallization rate model, accurately describes the crystallization mechanism, and improves the accuracy of the determined nuclei count.

The calculation formula for the supersaturation degree S is:

$$S = c_{M^{2+}} * c_{OH}{}^{2} / Ksp_{M(OH)_2} ;$$

wherein $c_{M^{2+}}$ refers to a concentration of divalent ions in a solution after iron and aluminum removal; $c_{OH}$ refers to a concentration of hydroxide ions; $Ksp_{M(OH)_2}$ refers to solubility product of hydroxide precipitate.

In a specific embodiment of the present application, the crystallization rate model is:

$$J = J_0 \exp(\frac{-16\pi\gamma^3 \overline{V}^2}{3k_B R_g{}^2 T^3 (\ln S)^2})$$

$$J_0 = A \cdot \ln S$$

$$S = c_{M^{2+}} * c_{OH}{}^{2} / Ksp_{M(OH)_2}$$

$$A = \frac{1}{V} \cdot (\frac{N_A R_g T}{\gamma}) 0.5 D_{M^{2+}} (Ksp_{M(OH)_2})^{1/3} ;$$

wherein $J$ refers to crystallization rate, $1/(m^3 \cdot s)$; $J_0$ refers to maximum crystallization rate, $1/(m^3 \cdot s)$; $\gamma$ refers to surface energy of hydroxide precipitate, $J/m^2$; $\overline{V}$ refers to partial molar volume of hydroxide, $mol/m^3$; $k_B$ refers to Boltzmann's constant; $R_g$ refers to ideal gas constant, $J/(mol \cdot K)$; $T$ refers to temperature, K; $N_A$ refers to Avogadro's constant; and $D_{M2+}$ refers to diffusion rate constant of $M^{2+}$ metal ions in a solution.

It should be noted that step S203 specifically involves: determining the nuclei count based on the crystallization rate, crystallization time, and the volume of the solution after iron and aluminum removal.

In some embodiments of the present application, as shown in FIG. 3, step S102 includes:

S301: determining a hydroxide precipitation concentration during primary growth of the crystal nuclei based on the primary growth reaction conditions; and

S302: determining the average particle size of the multiple crystal grains based on the hydroxide precipitation concentration, a partial differential equation for crystal grain growth, and a boundary condition.

The embodiment of the present application determines the influencing parameter of the primary growth reaction condition on the primary growth process of crystal nuclei, thus determines the average particle size of multiple grains generated after the primary growth reaction based on these influencing parameters, which ensures the accuracy of the average particle size under the primary growth reaction condition.

In a specific embodiment of the present application, the partial differential equation for crystal grain growth is as follows:

$$\frac{\partial C_{M(OH)_2}}{\partial t} = D\frac{\partial^2 C_{M(OH)_2}}{\partial r^2} + \frac{2D}{r}\frac{\partial C_{M(OH)_2}}{\partial r} \; ;$$

and the boundary condition is as follows:

$$C_{M(OH)_2}\frac{dR}{dt} = -D\frac{\partial C_{M(OH)_2}}{\partial r}\Big|_{r=R}$$

$$C_{M(OH)_2}(r = R) = \breve{C}_{M(OH)_2} \; ;$$

wherein $C_{M(OH)_2}$ refers to a concentration of hydroxide precipitate after the primary growth reaction condition; D refers to diffusion rate constant of hydroxide precipitate in a solution; r refers to vector position, t refers to time, c(r,t) refers to component field variable; R refers to maximum position of vector field; $\breve{C}_{M(OH)_2}$ refers to a concentration of hydroxide precipitate in the solution phase.

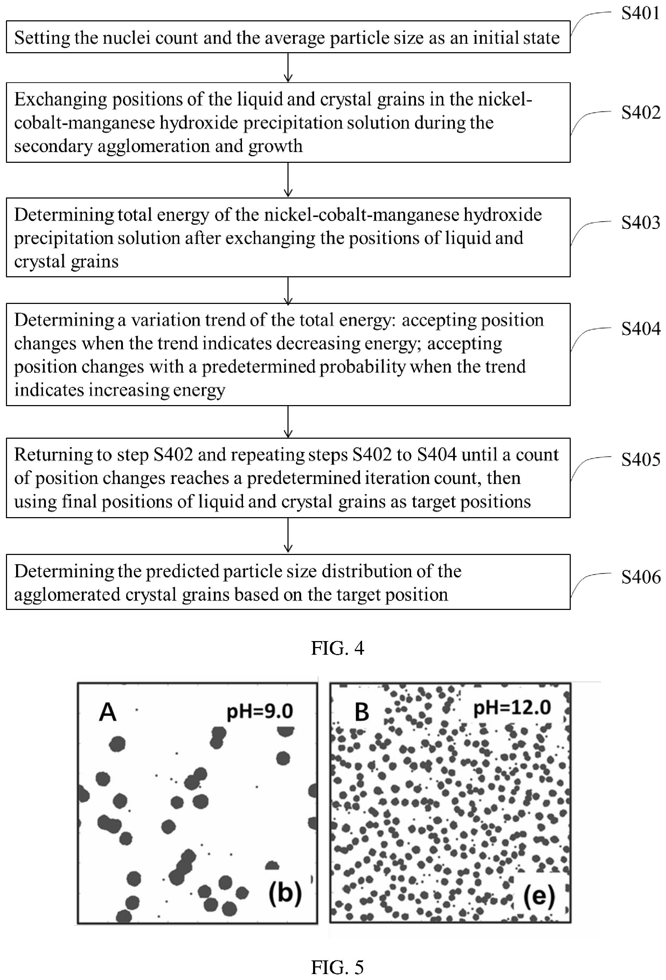

Since the lowest global energy situation is considered the most stable situation for the secondary aggregation and growth, that is: the optimal possible particle size distribution. Therefore, in some embodiments of the present application, as shown in FIG. 4, step S103 includes:

S401: setting the nuclei count and the average particle size as an initial state;

S402: exchanging positions of the liquid and crystal grains in the nickel-cobalt-manganese hydroxide precipitation solution during the secondary agglomeration and growth;

S403: determining total energy of the nickel-cobalt-manganese hydroxide precipitation solution after exchanging the positions of liquid and crystal grains;

S404: determining a variation trend of the total energy: accepting position changes when the trend indicates decreasing energy; accepting position changes with a predetermined probability when the trend indicates increasing energy;

S405: returning to step S402 and repeating steps S402 to S404 until a count of position changes reaches a predetermined iteration count, then using final positions of liquid and crystal grains as target positions; and

S406: determining the predicted particle size distribution of the agglomerated crystal grains based on the target position.

The embodiment of the present application by setting that if the change trend indicates a decrease, accepting the position change, and if the change trend indicates an increase, accepting the position change with a preset probability, which can ensure the process of predicting particle size distribution does not get trapped in local minimum value, further improving the accuracy of the predicted particle size distribution.

In some embodiments of the present application, the total energy is the energy between solid-liquid, which includes agglomeration energy and stirring energy. Specifically, the total energy is:

$$\varepsilon_{sl} = \frac{E_{agi} + E_{agglo}}{|E_{agi}|}$$

$$E_{agglo} = 4\pi(R_i(t))^2 \cdot \gamma$$

$$E_{agi} = -A_f \cdot \Omega \quad ;$$

wherein $\varepsilon_{sl}$ refers to total energy; $E_{agglo}$ refers to agglomeration energy; $E_{agi}$ refers to stirring energy; $R_i(t)$ refers to particle size of the particles after the secondary agglomeration at time t, m; $\gamma$ refers to surface energy density, mJ/m²; $A_f$ refers to proportional coefficient; and $\Omega$ refers to stirring speed, Hz.

It should be noted that during the exchange of the positions of the liquid and the grains in step S402, the distance between the liquid and grains should not be too far apart, and the maximum distance should be met:

$$\Delta x_{max} = \sqrt{D_p \times \Delta t}$$

wherein, $\Delta x_{max}$ refers to the maximum distance; $D_p$ refers to the diffusion rate of grains, m²/s; $\Delta t$ refers to the time interval between two consecutive iterative calculations.

The embodiment of the present application constrains the distance between the liquid and grains during the exchange of their positions, the process can be more consistent with the process of the secondary agglomeration and growth of grains, thereby further ensuring the accuracy of the predicted particle size distribution obtained.

In order to achieve quality control of the final obtained nickel-cobalt-manganese hydroxide product, some embodiments of the present application further include the following after step S103:

if the predicted particle size distribution does not meet the desired particle size distribution, optimize the nucleation reaction condition and/or the primary growth reaction condition until the predicted particle size distribution meets the desired particle size distribution.

By optimizing the conditions for nucleation reaction and/or primary growth reaction, the obtained predicted particle size distribution that meets the desired particle size distribution can be achieved, thus the control of the preparation of MHP via controlled crystallization in hydrometallurgical processing of laterite nickel ore can be achieved, and improving the quality of the obtained MHP product.

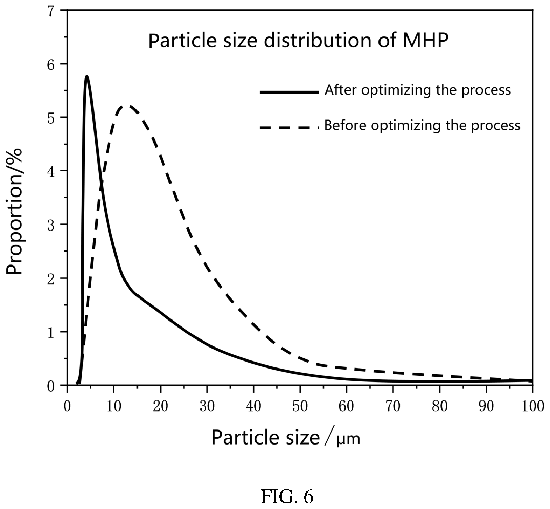

In a specific embodiment of the present application, FIG. 5 shows the predicted particle size distribution obtained in a case where the pH of the precipitating agent was 9.0 and 12.0, respectively; from FIG. 5, it can be seen that the higher the pH of the solution during precipitation, the smaller the average particle size of the final obtained product, which is more conducive to the subsequent filtration treatment. After adjusting the actual production process conditions according to the calculation results, the particle size distribution of the obtained MHP product is shown in FIG. 6. From FIG. 6, it can be seen that the MHP particle size distribution curve after process optimization has narrowed significantly and the overall particle size has decreased, which is consistent with the calculated prediction results, verifying the feasibility of this method.

An embodiment of the present application further provides a process for preparing MHP via controlled crystallization in hydrometallurgical processing of laterite nickel ore, which is achieved based on the method for preparing MHP via controlled crystallization in hydrometallurgical processing of laterite nickel ore;

wherein the method for preparing MHP via controlled crystallization in hydrometallurgical processing of laterite nickel ore is based on the method for preparing MHP via controlled crystallization in hydrometallurgical processing of laterite nickel ore as described in any one of the above embodiments.

Those skilled in the art should understand that the embodiment of all or part of the processes of method in the above embodiments can be accomplished by instructing the relevant hardware (such as a processor, a controller, etc.) through a computer program, which can be stored in a computer-readable storage medium. Among them, the computer-readable storage medium includes a disk, an optical disc, a read-only memory, or a random access memory, etc.

The above provides a detailed illustration on a method and a process for preparing MHP via controlled crystallization in hydrometallurgical processing of laterite nickel ore; specific examples are used herein to illustrate the principles and embodiment of the present application; the descriptions of the above examples are merely used to help understanding the methods and core ideas of the present application; simultaneously, for those skilled in the art, changes may occur in embodiment and application scope according to the ideas of the present application. In summary, the content of the specification should not be understood as a restriction on the present application.

**Claims**

1. A method for preparing MHP via controlled crystallization in hydrometallurgical processing of laterite nickel ore, comprising:

   determining a nuclei count of multiple crystal nuclei after nucleation reaction of a solution after iron and aluminum removal, based on nucleation reaction conditions;
   determining an average particle size of multiple crystal grains after primary growth of the multiple crystal nuclei, based on primary growth reaction conditions;
   determining a predicted particle size distribution of agglomerated crystal grains after secondary agglomeration and growth of the multiple crystal grains, based on the Metropolis algorithm, the nuclei count, and the average particle size.

2. The method for preparing MHP via controlled crystallization in hydrometallurgical processing of laterite nickel ore according to claim 1, wherein the nucleation reaction condition comprises pH and mass of a precipitating agent; wherein the determining the nuclei count of multiple crystal nuclei after nucleation reaction of a solution after iron and aluminum removal based on nucleation reaction conditions comprises:

   determining a supersaturation degree of a primary nickel-cobalt-manganese hydroxide precipitation solution based on the pH and mass of the precipitating agent;
   determining a crystallization rate of the primary nickel-cobalt-manganese hydroxide precipitation solution based on the supersaturation degree and a crystallization rate model;
   determining the nuclei count of the multiple crystal nuclei based on the crystallization rate.

3. The method for preparing MHP via controlled crystallization in hydrometallurgical processing of laterite nickel ore according to claim 2, wherein the crystallization rate model is as follows:

$$J = J_0 \exp(\frac{-16\pi\gamma^3 \overline{V}^2}{3k_B R_g^2 T^3 (\ln S)^2})$$

$$J_0 = A \cdot \ln S$$

$$S = c_{M^{2+}} * c_{OH}^2 / Ksp_{M(OH)_2}$$

$$A = \frac{1}{V} \cdot (\frac{N_A R_g T}{\gamma}) 0.5 D_{M^{2+}} (Ksp_{M(OH)_2})^{1/3};$$

   wherein $J$ refers to crystallization rate, $1/(m^3 \cdot s)$; $J_0$ refers to maximum crystallization rate, $1/(m^3 \cdot s)$; $\gamma$ refers to surface energy of hydroxide precipitate, $J/m^2$; $\overline{V}$ refers to partial molar volume of hydroxide, $mol/m^3$; $k_B$ refers to Boltzmann's constant; $R_g$ refers to ideal gas constant, $J/(mol \cdot K)$; $T$ refers to temperature, K; $S$ refers to supersaturation degree; $A$ refers to proportional constant; $c_{M2+}$ refers to a concentration of divalent ions in a solution after iron and aluminum removal; $c_{OH}$ refers to concentration of hydroxide ions; $Ksp_{M(OH)2}$ refers to solubility product of hydroxide precipitate; $N_A$ refers to Avogadro's constant; and $D_{M2+}$ refers to diffusion rate constant of $M^{2+}$ metal ions in a solution.

4. The method for preparing MHP via controlled crystallization in hydrometallurgical processing of laterite nickel ore according to claim 1, wherein the determining the average particle size of multiple crystal grains after primary growth of the multiple crystal nuclei based on primary growth reaction conditions comprises:

   determining a hydroxide precipitation concentration during primary growth of the crystal nuclei based on the primary growth reaction conditions;
   determining the average particle size of the multiple crystal grains based on the hydroxide precipitation concentration, a partial differential equation for crystal grain growth, and a boundary condition.

5. The method for preparing MHP via controlled crystallization in hydrometallurgical processing of laterite nickel ore according to claim 4, wherein the partial differential equation for crystal grain growth is as follows:

$$\frac{\partial C_{M(OH)_2}}{\partial t} = D\frac{\partial^2 C_{M(OH)_2}}{\partial r^2} + \frac{2D}{r}\frac{\partial C_{M(OH)_2}}{\partial r}\;;$$

and the boundary condition is as follows:

$$C_{M(OH)_2}\frac{dR}{dt} = -D\frac{\partial C_{M(OH)_2}}{\partial r}\Big|_{r=R}\;;$$

$$C_{M(OH)_2}(r = R) = \breve{C}_{M(OH)_2}\;;$$

wherein $C_{M(OH)2}$ refers to a concentration of hydroxide precipitate after the primary growth reaction condition; D refers to diffusion rate constant of hydroxide precipitate in a solution; r refers to vector position, t refers to time, c(r,t) refers to component field variable; R refers to maximum position of vector field; and $\breve{C}_{M(OH)_2}$ refers to a concentration of hydroxide precipitate in the solution phase.

6. The method for preparing MHP via controlled crystallization in hydrometallurgical processing of laterite nickel ore according to claim 1, wherein the determining a predicted particle size distribution of agglomerated crystal grains after secondary agglomeration and growth of the multiple crystal grains based on the Metropolis algorithm, the nuclei count and the average particle size comprises:

   step 1: setting the nuclei count and the average particle size as an initial state;
   step 2: exchanging positions of the liquid and crystal grains in the nickel-cobalt-manganese hydroxide precipitation solution during the secondary agglomeration and growth;
   step 3: determining total energy of the nickel-cobalt-manganese hydroxide precipitation solution after exchanging the positions of liquid and crystal grains;
   step 4: determining a variation trend of the total energy: accepting position changes when the trend indicates decreasing energy; accepting position changes with a predetermined probability when the trend indicates increasing energy;
   step 5: returning to Step 2 and repeating Steps 2 to 4 until a count of position changes reaches a predetermined iteration count, then using final positions of liquid and crystal grains as target positions; and
   step 6: determining the predicted particle size distribution of the agglomerated crystal grains based on the target positions.

7. The method for preparing MHP via controlled crystallization in hydrometallurgical processing of laterite nickel ore according to claim 6, wherein the total energy comprises agglomeration energy and stirring energy.

8. The method for preparing MHP via controlled crystallization in hydrometallurgical processing of laterite nickel ore according to claim 6, wherein a maximum distance between the exchanged-position liquid and crystal grains satisfies:

$$\Delta x_{max} = \sqrt{D_p \times \Delta t}$$

wherein $\Delta x_{max}$ refers to the maximum distance; $D_p$ refers to the diffusion rate of crystal grains, $m^2/s$; $\Delta t$ refers to the time interval between two consecutive iterative calculations.

9. The method for preparing MHP via controlled crystallization in hydrometallurgical processing of laterite nickel ore according to claim 1, wherein the method further comprises:
   when the predicted particle size distribution fails to satisfy a desired particle size distribution, optimizing the nucleation reaction conditions and/or the primary growth reaction conditions until the predicted particle size distribution satisfies the desired particle size distribution.

10. A process for preparing MHP via controlled crystallization in hydrometallurgical processing of laterite nickel ore, which is achieved based on the method for preparing MHP via controlled crystallization in hydrometallurgical processing of laterite nickel ore;

wherein the method for preparing MHP via controlled crystallization in hydrometallurgical processing of laterite nickel ore is based on the method for preparing MHP via controlled crystallization in hydrometallurgical processing of laterite nickel ore according to any one of claims 1-9.

Determining a nuclei count of multiple crystal nuclei after nucleation reaction of a solution after iron and aluminum removal, based on nucleation reaction conditions — S101

Determining an average particle size of multiple crystal grains after primary growth of the multiple crystal nuclei, based on primary growth reaction conditions — S102

Determining a predicted particle size distribution of agglomerated crystal grains after secondary agglomeration and growth of the multiple crystal grains, based on the Metropolis algorithm, the nuclei count, and the average particle size — S103

FIG. 1

Determining a supersaturation degree of a primary nickel-cobalt-manganese hydroxide precipitation solution based on the pH and mass of the precipitating agent — S201

Determining a crystallization rate of the primary nickel-cobalt-manganese hydroxide precipitation solution based on the supersaturation degree and a crystallization rate model — S202

Determining the nuclei count of the multiple crystal nuclei based on the crystallization rate — S203

FIG. 2

Determining a hydroxide precipitation concentration during primary growth of the crystal nuclei based on the primary growth reaction conditions — S301

Determining the average particle size of the multiple crystal grains based on the hydroxide precipitation concentration, a partial differential equation for crystal grain growth, and a boundary condition — S302

FIG. 3

Setting the nuclei count and the average particle size as an initial state — S401

Exchanging positions of the liquid and crystal grains in the nickel-cobalt-manganese hydroxide precipitation solution during the secondary agglomeration and growth — S402

Determining total energy of the nickel-cobalt-manganese hydroxide precipitation solution after exchanging the positions of liquid and crystal grains — S403

Determining a variation trend of the total energy: accepting position changes when the trend indicates decreasing energy; accepting position changes with a predetermined probability when the trend indicates increasing energy — S404

Returning to step S402 and repeating steps S402 to S404 until a count of position changes reaches a predetermined iteration count, then using final positions of liquid and crystal grains as target positions — S405

Determining the predicted particle size distribution of the agglomerated crystal grains based on the target position — S406

FIG. 4

FIG. 5

FIG. 6

**TRANSLATION**

### INTERNATIONAL SEARCH REPORT

| International application No. |
| --- |
| **PCT/CN2024/122776** |

| | |
| --- | --- |
| **A. CLASSIFICATION OF SUBJECT MATTER** | |
| C22B23/00(2006.01)i; G16C60/00(2019.01)i | |
| According to International Patent Classification (IPC) or to both national classification and IPC | |

**B. FIELDS SEARCHED**

Minimum documentation searched (classification system followed by classification symbols)

IPC: C22B23/-, G16C60/-

Documentation searched other than minimum documentation to the extent that such documents are included in the fields searched

Electronic data base consulted during the international search (name of data base and, where practicable, search terms used)

CJFD, CNTXT, ENTXTC, ENTXT, VEN: 格林美, 测算, 成核, 成核, 尺寸, 大小, 估测, 计算, 结晶, 晶核, 晶化, 粒径, 评估, 数量, 数目, 推测, 析晶, 预测, 预计, 预判, 预算, 直径, Budget, d50, Diameter, Evaluat+, metropolis, Number, Quantity, Size, Total, Estimat+, Nucleat+, Crystallizat+, Forecast+, Cryst., Measurement+, Anticipat+, Particle size, Crystal nucleus, Predict+, Calculat+

**C. DOCUMENTS CONSIDERED TO BE RELEVANT**

| Category* | Citation of document, with indication, where appropriate, of the relevant passages | Relevant to claim No. |
| --- | --- | --- |
| A | CN 117120642 A (PT QMB NEW ENERGY MATERIALS et al.) 24 November 2023 (2023-11-24)<br>  see description, paragraphs 6-10 | 1-10 |
| A | CN 106650008 A (SYSU-CMU SHUNDE INTERNATIONAL JOINT RESEARCH INSTITUTE et al.) 10 May 2017 (2017-05-10)<br>  entire document | 1-10 |
| A | CN 113192565 A (XI'AN UNIVERSITY OF TECHNOLOGY) 30 July 2021 (2021-07-30)<br>  entire document | 1-10 |
| A | CN 116822328 A (CENTRAL SOUTH UNIVERSITY) 29 September 2023 (2023-09-29)<br>  entire document | 1-10 |
| A | CN 118692599 A (YANSHAN UNIVERSITY) 24 September 2024 (2024-09-24)<br>  entire document | 1-10 |
| A | US 2023083184 A1 (IBM) 16 March 2023 (2023-03-16)<br>  entire document | 1-10 |

☐ Further documents are listed in the continuation of Box C.  ☑ See patent family annex.

| | |
| --- | --- |
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| **09 May 2025** | **15 May 2025** |

| Name and mailing address of the ISA/CN | Authorized officer |
| --- | --- |
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**
**Information on patent family members**

International application No.

**PCT/CN2024/122776**

| Patent document cited in search report | | | Publication date (day/month/year) | Patent family member(s) | | | Publication date (day/month/year) |
|---|---|---|---|---|---|---|---|
| CN | 117120642 | A | 24 November 2023 | WO | 2025000492 | A1 | 02 January 2025 |
| | | | | EP | 4516948 | A1 | 05 March 2025 |
| | | | | AU | 2023446716 | A1 | 30 January 2025 |
| CN | 106650008 | A | 10 May 2017 | CN | 106650008 | B | 20 March 2020 |
| CN | 113192565 | A | 30 July 2021 | None | | | |
| CN | 116822328 | A | 29 September 2023 | None | | | |
| CN | 118692599 | A | 24 September 2024 | None | | | |
| US | 2023083184 | A1 | 16 March 2023 | None | | | |

Form PCT/ISA/210 (patent family annex) (July 2022)